(19) Europäisches Patentamt
European Patent Office
Office européen des brevets

(11) Veröffentlichungsnummer : **0 150 418**
**B1**

(12) # EUROPÄISCHE PATENTSCHRIFT

(45) Veröffentlichungstag der Patentschrift : **04.03.87**

(51) Int. Cl.⁴ : **C 07 C 17/22**, C 07 C 79/12, C 07 C 76/02

(21) Anmeldenummer : **84115641.7**

(22) Anmeldetag : **17.12.84**

(54) **Verfahren zur Herstellung fluorsubstituierter aromatischer Verbindungen.**

(30) Priorität : **07.01.84 DE 3400418**

(43) Veröffentlichungstag der Anmeldung : **07.08.85 Patentblatt 85/32**

(45) Bekanntmachung des Hinweises auf die Patenterteilung : **04.03.87 Patentblatt 87/10**

(84) Benannte Vertragsstaaten : **BE CH DE FR GB IT LI NL**

(56) Entgegenhaltungen :
**DE-A- 2 938 939**
**US-A- 4 120 904**
**THE JOURNAL OF THE AMERICAN CHEMICAL SOCIETY, Band 78, 1956, Easton. G. C. FINGER et al.: "Aromatic Fluorine Compounds. VII. Replacement of Aromatic -Cl and -NO2 Groups by -F". Seiten 6034-6037**
**TETRAHEDRON LETTERS, 1978, Oxford. S. E. MORGAN et al.: "Some unexpected products form attempted halogen exchange in 2-nitrohaloaromatic systems". Seiten 4837-4838**

(73) Patentinhaber : **BASF Aktiengesellschaft**
**Carl-Bosch-Strasse 38**
**D-6700 Ludwigshafen (DE)**

(72) Erfinder : **Effenberger, F., Prof.-Dr.-Chem**
**Schatzweg 5**
**D-7000 Stuttgart 70 (DE)**
Erfinder : **Streicher, Willi**
**Andreas/Gryphibus-Strasse 7**
**D-5000 Köln 80 (DE)**

**EP 0 150 418 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Einführung des Fluoratoms in aromatische Verbindungen gehört zu den problematischen Operationen der organischen Chemie. Die bekannten Methoden — z. B. die Einführung über eine Diazoniumverbindung nach Balz-Schiemann — sind vom Standpunkt der industriellen Nutzung unbefriedigend.

Eine der bisher benutzten Methoden, nämlich der Ersatz der Nitrogruppe durch Fluor mit Hilfe von Salzen der Fluorwasserstoffsäure, z. B. Kaliumfluorid ist unbefriedigend hinsichtlich der Ausbeute (vgl. Morgan et al, Tetrah. Lett. 1978, 4837 ; Finger et al, JACS *78*, 6034 (1956).

Eine Möglichkeit, ausgehend von 2-Chlornitrobenzol mit Hilfe eines Alkalimetallfluorids 2-Fluornitrobenzol zu erhalten, ist u. a. aus der DE-AS 29 38 939 bekannt, wobei Sulfolan als Lösungsmittel verwendet und ein Phasentransfer-Katalysator zugesetzt wird. Nach diesem Verfahren kann jedoch nicht die Nitrogruppe durch Fluor ersetzt werden.

Es wurde nun gefunden, daß der Ersatz der aromatisch gebundenen Nitrogruppe durch Fluor, d. h. die Herstellung fluorsubstituierter aromatischer Verbindungen durch Umsetzung der entsprechenden Nitroverbindung mit einem Salz der Fluorwasserstoffsäure mit guter Ausbeute in Gegenwart von Säurehalogeniden gelingt.

In der Regel tritt eine Verbesserung von Umsatz und Ausbeute bei allen, z. B. von Finger et al beschriebenen Umsetzungen ein. Nitroverbindungen, die eine aktivierte Nitrogruppe enthalten, verhalten sich besonders günstig. So eignen sich für das Verfahren z. B. Halogennitrobenzole, m-Dinitrobenzole und besonders Trinitrobenzole. Von letzteren sind eine oder zwei Nitrogruppen durch Fluor ersetzbar. Quasiaromatische Verbindungen wie z. B. Nitropyridine sind ebenfalls umsetzbar.

Als Salze der Fluorwasserstoffsäure sind vor allem die Alkalisalze geeignet, aus wirtschaftlichen Gründen bevorzugt man Kaliumfluorid.

Als Säurehalogenid bevorzugt man aus wirtschaftlichen Gründen Säurechloride. Man kann z. B. aromatische Carbonsäurehalogenide wie Phthaloylchlorid verwenden ; wegen der meist erforderlichen Reaktionstemperatur von etwa 150 bis 250 °C sind aliphatische Carbonsäurehalogenide weniger geeignet. Andererseits können, wenn geeignete Apparaturen zur Verfügung stehen, z. B. Phosgen, Thionylchlorid oder Oxalylchlorid verwendet werden. In diesen Fällen arbeitet man zweckmäßig unter überatmosphärischem Druck.

Die Umsetzung sollte, um hinreichende Löslichkeit der Reaktionsteilnehmer zu erzielen, in einem, evtl. hochsiedenden polar-aprotischen Lösungsmittel vorgenommen werden. Geeignet sind z. B. Stoffe wie Hexamethylphosphorsäuretriamid, Dimethylformamid, N-Methylpyrrolidon oder, besonders, Sulfolan. Wenn ein Reaktionsraum für die Arbeiten unter erhöhtem Druck zur Verfügung steht, können auch niedriger siedende Lösungsmittel benutzt Mu/P werden. In manchen Fällen eignet sich das Reaktionsprodukt selbst als Lösungsmittel.

Bei der Bemessung der Mengen an Reaktionsteilnehmern ist zu berücksichtigen, daß vermutlich dem Austausch der Nitrogruppe gegen Fluor ein Austausch des Halogenatoms der Säure gegen Fluor vorausgeht und die austretende Nitrogruppe in Form eines Nitritions mit einem als Säurehalogenid gebundenen Fluoratoms unter Bildung von Nitrosylfluorid reagiert. Je Äquivalent umzusetzender Nitrogruppen sollten demnach 2 Äquivalente Fluorid und 2 Äquivalente Säurehalogenid zur Verfügung stehen. Die Verwendung eines Überschusses an dem einen oder anderen Reaktionsteilnehmer richtet sich nach wirtschaftlichen Überlegungen.

Die vorstehenden Überlegungen sind rein theoretischer Art und sollen die Erfindung in keiner Weise begrenzen.

Beispiel 1

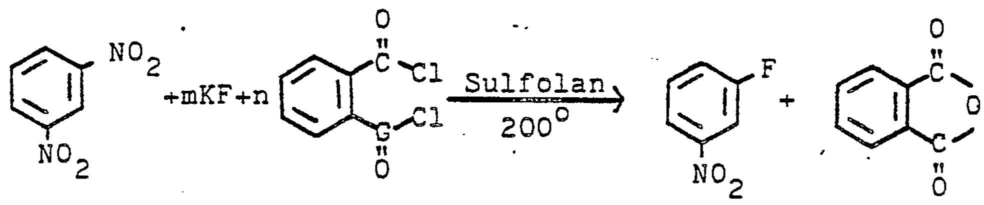


32,50 g (193, 45 mmol) 1,3-Dinitrobenzol (1,3-DNB)
56,10 g (967,2 mmol) KF
57,6 g (283,34 mmol) Phthaloylchlorid (PC)
200 ml Sulfolan

Durchführung der Reaktion

32,50 g 1,3-DNB, 56,10 g KF und 39,27 g Phthaloylchlorid werden gemeinsam in 200 ml Sulfolan auf 200 °C erwärmt. Nach 24 Stunden werden 10 ml Phthaloylchlorid bei 140 °C zugesetzt und 15 Minuten bei dieser Temperatur gerührt, dann weiter auf 200 °C erwärmt. Weitere Zugabe von 3 ml Phthalochlorid nach 46 Stunden bei 140° ; es wird 15 Minuten bei dieser Temperatur gerührt, dann auf 200 °C erwärmt. Das Reaktionsgemisch wird unter vermindertem Druck (Ölpumpe) destilliert.

1. Fraktion : 15,27 g 3-Nitrofluorbenzol (gaschromatographisch einheitlich)

2. Fraktion : Das Destillat wird mit Ether aufgenommen und mit Wasser lösungsmittelfrei gewaschen. Der Etherextrakt wird über Magnesiumsulfat getrocknet und im Dünnschichtverdampfer konzentriert. Es werden noch 3,82 g reines 3-Nitrofluorbenzol gewonnen. Gesamtausbeute (135 mmol) 70 %, bezogen auf Dinitrobenzol.

3. Fraktion : Die dritte Fraktion besteht aus Sulfolan und Phthalsäureanhydrid. Das Phthalsäureanhydrid wird mit verdünnter Salzsäure gefällt, abgesaugt, mit Wasser nachgewaschen und getrocknet. Ausbeute 30,0 g (78 %) Phthalsäureanhydrid.

Beispiel 2

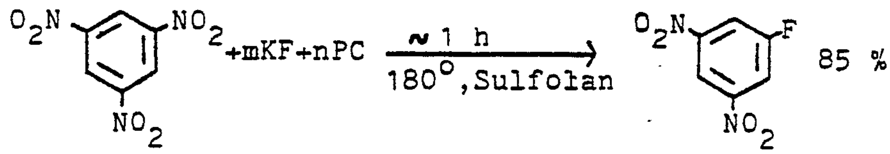


6,18 g (29,01 mmol) 1,3,5 Trinitrobenzol
8,41 (145,07 mmol) KF
5,89 g (29,01 mmol) Phthaloylchlorid
19,50 ml Sulfolan

Umsetzungsdauer :
53 Minuten

Aufarbeitung :
Das Reaktionsgemisch wird mit Wasser verdünnt und mit Ether aufgenommen. Dann wird mit Wasser gewaschen, über Magnesiumsulfat getrocknet und der Ether im Dünnschichtverdampfer entfernt. Der Rückstand wird in Methylenchlorid gelöst und über Kieselgel chromatographiert.

Ausbeute :
4,85 g = 85,0 % 3,5-Dinitrofluorbenzol. Schmelzpunkt 43 °C, entsprechend dem bekannten Wert (43,8 °C ; Bye, J. Chem. Soc. 1954, 3091).

Beispiel 3

Ansatz :
15,00 g (70,04 mmol) 1,3,5-Trinitrobenzol
28,59 g (493,95 mol) KF
38,59 g (140,84 mmol) Phthaloylchlorid
47,00 ml Sulfolan

Umsetzungsdauer :
12 Stunden ; Ausbeute nach Destillation : 7,84 g = 70 % 3,5-Difluornitrobenzol.

Beispiel 4

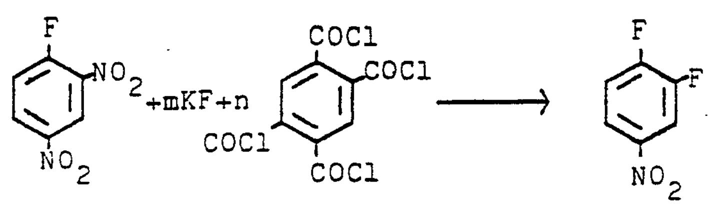


Ansatz :
1,80 g (9,68 mmol) 2,4-DNFB

3,15 g (9,66 mmol) Pyromellithsäuretetrachlorid
3,37 g (59,10 mmol) KF
13 ml Sulfolan

Man verfährt, wie vorstehend beschrieben (200 °C, 4 Stunden).

Ausbeute :
3,4-Difluornitrobenzol : 58 %
nicht umgesetztes 2,4-Dinitrofluorbenuol : 11 %.

**Patentansprüche**

1. Verfahren zur Herstellung fluorsubstituierter aromatischer Verbindungen durch Umsetzung der entsprechenden Nitroverbindung mit einem Salz der Fluorwasserstoffsäure, dadurch gekennzeichnet, daß man die Umsetzung in Gegenwart eines Säurehalogenids vornimmt.
2. Verfahren nach Anspruch 1, dadurch gekennzeichnet, daß man die Umsetzung in Gegenwart eines Carbonsäurehalogenids vornimmt.
3. Verfahren nach Anspruch 2, dadurch gekennzeichnet, daß man die Umsetzung in Gegenwart von Phthaloylchlorid vornimmt.
4. Verfahren nach Anspruch 1, dadurch gekennzeichnet, daß man die Umsetzung in einem polaren, aprotischen Lösungsmittel vornimmt.
5. Verfahren nach Anspruch 1, dadurch gekennzeichnet, daß man die Umsetzung in der Weise vornimmt, daß das Säurehalogenid dem Reaktionsgemisch zugeführt wird.

**Claims**

1. A process for the preparation of a fluorine-substituted aromatic compound by reacting the corresponding nitro compound with a salt of hydrofluoric acid, wherein the reaction is carried out in the presence of an acid halide.
2. A process as claimed in claim 1, wherein the reaction is carried out in the presence of a carboxylic acid halide.
3. A process as claimed in claim 2, wherein the reaction is carried out in the presence of phthaloyl chloride.
4. A process as claimed in claim 1, wherein the reaction is carried out in a polar aprotic solvent.
5. A process as claimed in claim 1, wherein the reaction is carried out by adding the acid halide to the reaction mixture.

**Revendications**

1. Procédé de préparation de composés aromatiques substitués par du fluor, par réaction du composé nitro correspondant avec un sel de l'acide fluorhydrique, caractérisé par le fait que l'on effectue la réaction en présence d'un halogénure d'acide.
2. Procédé selon la revendication 1, caractérisé par le fait que l'on effectue la réaction en présence d'un halogénure d'acide carboxylique.
3. Procédé selon la revendication 2, caractérisé par le fait que l'on effectue la réaction en présence d'un chlorure de phtaloyle.
4. Procédé selon la revendication 1, caractérisé par le fait que l'on effectue la réaction dans un solvant aprotique, polaire.
5. Procédé selon la revendication 1, caractérisé par le fait que l'on effectue la réaction en amenant l'halogénure d'acide au mélange de réaction.